(19) Europäisches Patentamt

European Patent Office

Office européen des brevets

(11) Publication number: **0 489 722 B1**

(12) **EUROPEAN PATENT SPECIFICATION**

(45) Date of publication of patent specification: **01.02.95** (51) Int. Cl.⁶: **C07C 209/16**

(21) Application number: **92200339.7**

(22) Date of filing: **07.10.88**

(60) Publication number of the earlier application in accordance with Art.76 EPC: **0 312 253**

(54) **Process for preparing N-substituted amine.**

(30) Priority: **16.10.87 JP 261366/87**
**16.10.87 JP 261367/87**
**16.10.87 JP 261368/87**
**16.10.87 JP 261369/87**

(43) Date of publication of application:
**10.06.92 Bulletin 92/24**

(45) Publication of the grant of the patent:
**01.02.95 Bulletin 95/05**

(84) Designated Contracting States:
**DE ES**

(56) References cited:
**DE-A- 3 034 433**
**DE-A- 3 641 666**
**US-A- 4 625 063**

(73) Proprietor: **KAO CORPORATION**
**14-10, Nihonbashi Kayaba-cho 1-chome**
**Chuo-ku**
**Tokyo (JP)**

(72) Inventor: **Abe, Hiroshi**
**1450 Nishihama**
**Wakayama-shi,**
**Wakayama (JP)**

Inventor: **Aikawa, Jun**
**1130, Nishihama**
**Wakayama-shi,**
**Wakayama (JP)**
Inventor: **Okabe, Kazuhiko**
**283-28, Hatage,**
**Iwadecho**
**Naga-gun,**
**Wakayama (JP)**
Inventor: **Sotoya, Kohshiro**
**446-26, Nakaguro,**
**Iwadecho**
**Naga-gun,**
**Wakayama (JP)**

(74) Representative: **Bannerman, David Gardner et al**
**Withers & Rogers**
**4 Dyer's Buildings**
**Holborn**
**London, EC1N 2JT (GB)**

**Description**

The present invention relates to a process for preparing an N-substituted amine which comprises reacting an alcohol or an aldehyde with ammonia or a primary or secondary amine.

The amine prepared according to the present invention is a material important from the industrial viewpoint as intermediates of a rust preventive, a surfactant, a germicide, a dyeing assistant and a fiber softener, etc.

[Prior Art]

It is known in the art that an alcohol or an aldehyde is reacted with ammonia or a primary or secondary amine to give the corresponding amine. However, it was difficult to selectively prepare a particular amine through a reaction of an alcohol or the like with an amine or the like.

Examples of the process for preparing an amine from an alcohol and an amine include those disclosed in Japanese Patent Laid-Open Nos. 19604/1977 (a copper chromite catalyst and a cobalt catalyst) and 59602/1978 (copper/molybdenum and copper/tungsten catalysts), U.S. Patent No. 3 223 734 (Raney nickel catalyst and copper chromite catalysts), West German Patent Laid-Open No. 1,493,781 (a supported cobalt catalyst), Japanese Patent Publication No. 55704/1982 (a copper/nickel catalyst), etc. However, these catalysts are insufficient in both the catalytic activity and the selectivity and should be used in a large amount, which brings about a low yield of the intended amine. In order to solve these problems, processes described in Japanese Patent Laid-Open Nos. 15865/1986, 149646/1987, 149647/1987, and 149648/1987 have been developed. In these processes, a copper/nickel/group VIII platinum metal element catalyst is used to prepare an intended amine in a high yield. That is, in these processes, an intended amine is prepared in a high yield through the addition of a small amount of the group VIII platinum metal element to a conventional copper/nickel catalyst having insufficient activity and selectivity for the purpose of improving the activity and selectivity.

However, the process in which the reaction is conducted in the presence of this catalyst is not always satisfactory from the viewpoint of the durability of the catalyst. Specifically, although this process is superior in the activity and the selectivity to other general processes and brings about little or no lowering in the activity of the catalyst even after recovery and reuse of tens of times, the selectivity is lowered. Therefore, with the consideration of the preparation of an N-substituted amine on a commercial scale, a further improvement in the durability is desired from the viewpoint of the yield and quality of the formed N-substituted amine.

However, N-substituted amines which have been prepared in the presence of these catalysts bring about the deterioration of hue when converted into a quaternary ammonium salt (tetraalkylammonium salt, trialkylbenzylammonium salt, etc.), which unfavorably causes remarkable deterioration of performance in applications such as surfactants.

Other documents which disclose the production of amines using catalysts are US-A-4625063, DE-A-3641666 and DE-A-3034433. US-A-4625063 and DE-A-3641666 disclose the reaction of an alcohol or aldehyde with a primary amine in the presence of a copper/nickel/group-VIII noble metal catalyst. DE-A-3034433 discloses the production of tertiary monoamines and polyamines by reacting various alcohols, ketones, aldehydes or ethylene oxide with ammonia, or a primary or secondary aliphatic amine, in the presence of a catalyst containing copper or silver carboxylate; group VIII, manganese or zinc carboxylate; and fatty acid, alkali metal or alkaline earth metal carboxylates.

(Summary of the Invention)

The invention provides a process for preparing an N-substituted amine, which comprises the step of reacting an alcohol or an aldehyde with ammonia, a primary amine or a secondary amine at 150 to 250°C at a pressure of the atmospheric pressure to 100 atm gauge, while water formed in the reaction is being removed out, in the presence of a catalyst comprising:

(1) copper, (2) a metal selected from the group consisting of chromium, manganese, iron, cobalt, nickel and zinc, (3) a metal of the platinum VIII group and (4) a metal selected from the group consisting of alkali metals and alkaline earth metals.

It is preferable that a molar ratio of the component (1) to (2) ranges from 10/90 to 99/1, preferably 1/9 to 9/1 or 50/50 to 99/1, a molar ratio of the component (3) to the sum total of (1) and (2) ranges from 0.001 to 0.1 and a molar ratio of the component (2) to (4) ranges from 1/0.01 to 1/1.

The invention will be explained in detail in with reference to the catalyst.

It has been found that the addition of a small amount of a fourth component metal element composed of an alkali or alkaline earth metal selected from among lithium, sodium, potassium, rubidium, cesium, magnesium, calcium, strontium, and barium to the copper/fourth period transition metal element/group VIII platinum metal element catalyst brings about a remarkable improvement in the selectivity while substantially maintaining the activity. At the same time, the present inventors have found that the quaternary ammonium salt prepared through the conversion of the starting material composed of the N-substituted amine prepared in the presence of this catalyst has a remarkably improved hue over that of the conventional quaternary ammonium salt. In this case, chromium, manganese, iron, cobalt, nickel, and zinc were useful as the fourth period transition metal element while platinum, palladium, ruthenium, and rhodium were useful as the group VIII platinum metal element.

Consequently, the addition of a small amount of a fourth component metal element composed of an alkali or alkaline earth metal to a copper/fourth period transition metal element/group VIII platinum metal element catalyst enabled the development of a high-performance amination catalyst not only having activity equal to that of the copper/fourth period transition metal element/group VIII platinum metal element catalyst and selectivity far superior to that of the copper/fourth period transition metal element/group VIII platinum metal element catalyst but also capable of preparing an N-substituted amine which can be converted into a quaternary ammonium salt having very excellent hue.

It is necessary that the catalyst used in the present invention contain copper, a fourth period transition metal element, a group VIII platinum metal element (hereinafter abbreviated to "platinum group element"), and a fourth metal element (hereinafter referred to as "fourth component"). In the catalyst metal composition used, the proportions of copper, the fourth period transition metal element, the platinum group element, and the fourth component may be arbitrary. However, the molar ratio of copper to the fourth period transition metal element is preferably 10 : 90 to 99 : 1, more preferably 50 : 50 to 99 : 1. The ratio of amount of addition of the platinum group element to the total of copper and the fourth period transition metal element is preferably 0.001 to 0.1 (molar ratio), more preferably 0.001 to 0.05. Further the molar ratio of the fourth period transition metal element to the fourth component is preferably 1 : 0.01 to 1 : 1, more preferably 1 : 0.01 to 1 : 0.5.

The fourth period transition metal elements particularly suitable for the reaction according to the present invention are chromium, manganese, iron, cobalt, nickel, and zinc. The platinum group elements particularly suitable for the reaction according to the present invention are platinum, palladium, ruthenium, and rhodium. Further, the fourth component particularly useful for the reaction according to the present invention includes lithium, sodium, potassium, rubidium, cesium, magnesium, calcium, strontium, and barium.

Although the catalyst metal composition should contain four components, i.e., copper, a fourth transition metal element, a platinum group element, and a fourth component, the catalyst suitable for the present invention may have various forms.

Specifically, in the present invention, only when the four components, i.e., copper, a fourth period transition metal element, a platinum group element, and a fourth component are present in the reaction system as the catalyst composition, an effect can be attained through interaction among the four components. Therefore, this four component composition has a substantial function as a catalyst, and in the reaction the catalytic activity is not developed until each metallic component is reduced in a hydrogen atmosphere. For this reason, in the present invention, there is no limitation with respect to the form of the metals before the reduction and the state of the system after the completion of the reduction as far as the reduction in a hydrogen atmosphere according to the method described in the present specification brings about interaction among copper, the fourth period transition metal element, the platinum group element, and the fourth component.

Therefore, the metals suitable for the process of the present invention may have any of the following forms as far as the four metal components indispensable to the present invention bring about interaction thereamong through reduction in a hydrogen atmosphere:

1) a form of a metal, an oxide or a hydroxide thereof and a mixture thereof which can be dispersed in a reaction medium;

2) a form of either a mixture of copper, a fourth period transition metal element, a platinum group element and a fourth component respectively supported on suitable carriers, or three components, i.e., copper, a fourth period transition metal element, a platinum group element, and a fourth component, homogeneously supported on a single carrier which can be dispersed in a reaction medium;

3) a form of an aliphatic carboxylic acid salt of these metals, a complex of these metals stabilized by a suitable ligand or the like which is converted into a metallic colloid in a reaction medium to form a homogeneous system; and

3

4) a mixture of a form described in the above items 1) and 2) which is dispersed in a reaction medium with a form described in the above item 3) which forms a homogeneous system in a reaction system, or a form which is in a dispersed state before hydrogen reduction but becomes homogeneous after hydrogen reduction.

With respect to the form of the catalyst used in the process of the present invention, it is preferred from the viewpoint of the stabilization of the catalyst metal, i.e., immobilization of the active surface, and resistance to a catalyst poison that the above-described four metal components are homogeneously supported on a suitable carrier.

When the four metal components according to the present invention, i.e., copper, a fourth period transition metal element, a platinum group element, and a fourth component, are to be supported on a carrier, suitable carriers are those commonly employed as catalyst carriers, e.g., alumina, silica/alumina, diatomaceous earth, silica, active carbon, and natural and artificial zeolite. Although the amount of the catalyst metal supported on a carrier may be arbitrarily determined, it is generally preferably 5 to 70%.

Further, these four metal components may be supported on a carrier by various methods. In this case, the form of the raw metals of the catalyst may be an oxide, a hydroxide, or various salts of copper, a fourth period transition metal element, and a platinum group element. Examples of the form of the raw metals include chlorides, sulfates, nitrates, acetates, aliphatic carboxylates of copper, a fourth period transition metal element, a platinum group element, and a fourth component, or complexes of these metals, e.g., acetylacetone complexes and dimethylglyoxime complexes of copper, a fourth period transition metal element, a platinum group element, and a fourth component. Further, with respect to the platinum group element, carbonyl complexes, amine complexes, phosphine complexes, etc. may also be employed. The preparation of a catalyst through supporting of the metal components on a carrier by making use of these raw metal materials may be conducted by any of conventional known processes, e.g., a process which comprises adding a carrier to a solution of suitable salts of copper, a fourth period transition metal element, a platinum group element, and a fourth component, to sufficiently impregnate the carrier with the solution and drying and baking the impregnated carrier (impregnation process), a process which comprises conducting either a step of sufficiently mixing a carrier with an aqueous solution of suitable salts of copper, a fourth period transition metal element and a platinum group element and then adding an aqueous alkaline solution, such as an aqueous sodium carbonate or sodium hydroxide solution or aqueous ammonia, to the mixture to precipitate the metal salts on the carrier, or a step or simultaneously adding an aqueous solution of suitable salts of copper, a fourth period transition metal element and a platinum group element and an aqueous alkaline solution, such as an aqueous sodium carbonate or sodium hydroxide solution or aqueous ammonia, to a water slurry of a carrier in such a manner that the pH value of the slurry remains constant (e.g., a constant pH value of 7) to precipitate the metal salts on the carrier, drying and baking the metal salts supported on the carrier to prepare a copper/fourth period transition metal element/platinum group element catalyst, placing the resulting ternary catalyst in an aqueous solution of an alkali metal salt or an alkaline earth metal salt to impregnate the catalyst with the aqueous solution, and drying and baking the impregnated catalyst (a combination of the coprecipitation process with the impregnation process), and a process which comprises conducting an ion exchange with hydrogen or a metal contained in zeolite (ion exchange process). In the case of the coprecipitation process, the carrier is sufficiently washed with water after deposition of the metals, dried at about 100°C, and baked at 300 to 700°C to prepare a catalyst.

An other effective process comprises supporting only copper or only copper and a fourth period transition metal element on a carrier through the above-described processes and adding, prior to the reaction, a supported platinum group element or fourth/component, or an aliphatic carboxylate or a complex thereof to form a composite comprising a combination of copper with the fourth period transition metal element, the platinum group element and the fourth component in a reaction medium in a hydrogen atmosphere.

With respect to the form of the catalyst, it is more preferable that the four components be homogeneously supported on the same carrier.

The four components, i.e., copper, a fourth period transition metal element, a platinum group element, and a fourth component, are indispensable to the present invention.

The alcohol or aldehyde which is a starting material used in the present invention is a straight-chain or branched saturated or unsaturated aliphatic alcohol or aldehyde having 8 to 36 carbon atoms. Examples of the alcohol include octyl alcohol, lauryl alcohol, myristyl alcohol, stearyl alcohol, behenyl alcohol, oleyl alcohol, and a mixture thereof, a Ziegler alcohol prepared by the Ziegler process, an oxo alcohol prepared by the oxo process, and alcohols having a branched chain such as Guerbet alcohol. Examples of the aldehyde include lauraldehyde, oxo aldehyde, and aldehydes corresponding to the above-described alcohols.

4

EP 0 489 722 B1

Further, various polyhydric alcohols may also be used, and examples thereof include 1,3-butanediol, 1,4-butanediol, 1,5-pentanediol, 1,6-hexanediol, 1,9-nonanediol, diethylene glycol, and triethylene glycol. Examples of other alcohol include aromatic alcohols, such as benzyl alcohol and phenethyl alcohol, polyoxy ether alcohols, such as adduct of an aliphatic alcohol with ethylene oxide or propylene oxide, and amino alcohols, such as ethanolamine and diethanolamine.

The alcohol or aldehyde is particularly preferably an aliphatic alcohol or aldehyde selected from among saturated and unsaturated straight-chain and branched aliphatic alcohols and aldehydes having 8 to 36 carbon atoms and aliphatic glycols having 2 to 12 carbon atoms. The amine which is reacted with these alcohols or aldehydes may be a gaseous or liquid one, and examples thereof include aliphatic amines, e.g., primary amines such as monomethylamine, ethylamine and dodecylamine, and secondary amines such as dimethylamine, diethylamine and didodecylamine.

In the present invention, it is necessary to remove water formed during a reaction of an alcohol or an aldehyde with an amine from the reaction system. When the formed water is not removed from the reaction system, no sufficient catalytic performance according to the present invention can be attained. That is, the catalytic activity and selectivity are lowered, so that it becomes difficult to easily prepare an N-substituted amine in a high yield. For example, when the reaction is conducted by making use of dimethylamine as the amine without removing the formed water, not only the amount of by-products, such as monoalkylmethylamine, which are difficult to separate through mere distillation is increased but also a high-boiling material, such as condensate of an aldehyde, is formed in a large amount, which brings about a lowering in the yield of the intended N-substituted amine.

Water may be intermittently or continuously removed during the reaction as far as the formed water is properly removed so as not to remain in the reaction system for a long period of time. It is preferred to continuously remove water each time it is formed. More specifically, it is a common practice to introduce a suitable amount of hydrogen gas into the reaction system to distill the formed water and the excess amine (in the case of use of a gaseous amine) together with the hydrogen gas. In this method, it is also possible to reuse the hydrogen gas through condensation and separation of the formed water in a condenser. Further, a suitable solvent may be added to the reaction system, followed by distillation and removal of the formed water in the form of an azeotrope with the solvent.

In the process of the present invention, although a catalyst which has separately been reduced with a hydrogen gas may be employed, a catalyst before reduction is placed in a reactor together with the starting material of the reaction, i.e., an alcohol or an aldehyde, followed by elevation of the temperature to the reaction temperature while introducing a hydrogen gas, thereby conducting reduction. That is, the copper/fourth period transition metal element/platinum group element catalyst according to the present invention has also a significant feature that since the reduction temperature is low, the reduction can be conducted in the step of elevating the temperature to the reaction temperature.

The preferred embodiments of the process of the present invention will now be briefly described.

A reaction vessel equipped with a tube for introducing hydrogen and an amine and a condenser and a separator for condensing and separating water formed during the reaction, the excess amine and oleaginous matter distilled off is charged with a starting material, i.e., an alcohol or an aldehyde, and a catalyst. The amount of the catalyst may be arbitrary. Since, however, the catalyst of the present invention has high activity, it is usually employed in an amount as small as 0.1 to 2 % by weight based on the feed alcohol or aldehyde. The system is purged with a nitrogen gas, and the temperature is then raised while introducing hydrogen. The reaction is usually conducted at a temperature of 150 to 250°c. However, the reaction temperature may be one outside the above-described range depending upon the kind of the reaction. The catalyst is converted into a reduced, active state during the elevation of the temperature. When the temperature reaches a predetermined value, ammonia or an amine is introduced to initiate the reaction. The amine may be gaseous or liquid. Further, the amine may be introduced into the system in a continuous or intermittent manner or at a time (in the case of a liquid amine). During the reaction, the formed water is discharged outside the reaction system together with gaseous substances (excess gaseous amine in the case of the use of hydrogen and a gaseous amine) and passed through the condenser and separator to separate the water from the oleaginous matter. The analysis of the gaseous substances (excess gaseous amine in the case of the use of hydrogen and a gaseous amine) has revealed that these gaseous substances are substantially free from by-products (e.g., hydrocarbon and amine by-product formed by disproportionation of the starting amine), i.e., has proved that the catalyst of the present invention has high selectivity. Therefore, it was found that these gaseous substances can be reused without any special step of purification. After the completion of the reaction, the reaction mixture itself is subjected to distillation or filtration to separate the reactant from the catalyst. The N-substituted amine obtained by the filtration can be distilled into a very pure form.

5

Examples of the Catalyst

Examples 1 and 2 and Comparative Examples 1 to 4

A copper/fourth period transition metal element/platinum group element/fourth component quaternary catalyst supported on synthetic zeolite was prepared as follows.

A 1-$\ell$ flask was charged with synthetic zeolite, and an aqueous solution prepared by dissolving copper nitrate, nickel nitrate and palladium chloride in water in a copper : nickel : palladium molar ratio of 4 : 1 : 0.05 was then added thereto, followed by elevation of the temperature while stirring. An aqueous 10% $Na_2CO_3$ solution was gradually dropwise added thereto at 90°C. The mixture was aged for 1 hr, and the resultant precipitates were collected by filtration, washed with water, dried at 80°C for 10 hr, and baked at 400°C for 3 hr. The resultant ternary catalyst was impregnated with an aqueous lithium carbonate solution (a Ni to Li molar ratio of 1 : 0.05) and again dried at 80°C for 10 hr, followed by baking at 300°C for 1 hr. The amount of the resultant metallic oxide supported on the carrier was 50%.

Then, a reaction of an alcohol with dimethylamine was conducted in the presence of the above-prepared catalyst. In Comparative Examples, the reaction was conducted in the presence of a copper/nickel/palladium catalyst and a copper/nickel catalyst prepared in the same manner as that described above.

A 1-$\ell$ flask equipped with a condenser and a separator for separating the formed water was charged with 600 g of lauryl alcohol and 1.5 g (0.25% by weight based on the starting alcohol) of the above-described catalyst. The system was purged with nitrogen while stirring, followed by initiation of the elevation of the temperature. When the temperature reached 100°C, a hydrogen gas was blown into the system through a flowmeter at a flow rate of 10 $\ell$/hr, and the temperature was raised to 200°C. At this temperature, a mixed gas comprising dimethylamine and hydrogen was blown into the reaction system at a flow rate of 40 $\ell$/hr, and the reaction was monitored by measurement of an amine value and gas chromatography.

The results are shown in Table 1.

It was found from the results that as with the Cu/Ni/platinum group element (Pd) ternary catalyst system, the Cu/fourth period transition metal element (Ni)/platinum group element (Pd)/fourth component (Li) four component catalyst system according to the present invention exhibited higher activity than that of the conventional Cu/Ni binary catalyst system (Comparative Example 1) and a remarkable improvement in the selectivity thereof.

Table 1

| | catalyst composition molar ratio | reaction time (hr) | composition of reaction product (wt%) | | |
|---|---|---|---|---|---|
| | | | unreacted alcohol | lauryldimethylamine | Others |
| Ex. 1 | Cu/Ni/Pd/Li = 4/1/0.05/0.05 | 5 | 1.6 | 97.6 | 0.8 |
| Comp. Ex. 1 | Cu/Ni = 4/1 | 10 | 5.3 | 89.0 | 5.7 |
| Comp. Ex. 2 | Cu/Ni/Pd = 4/1/0.05 | 5 | 1.8 | 92.3 | 5.9 |

Then, lauryldimethylamine prepared in the presence of the above-described catalysts was purified by distillation, followed by a reaction with benzyl chloride or methyl chloride under ordinary conditions to synthesize a quaternary ammonium salt of lauryldimethylamine. The hue of the quaternary ammonium salt thus prepared was measured with Lovibond Red (by making use of a 1-in. cell).

The results are shown in Table 2.

## Table 2

| | catalyst * composition | Lovibond Red | |
|---|---|---|---|
| | | trimethyllauryl- ammonium chloride | dimethyllaurylbenzyl- ammonium chloride |
| Ex. 2 | Cu/Ni/Pd/Li | 0.8 | 0.6 |
| Comp. Ex. 3 | Cu/Ni | 5.0 | 4.5 |
| Comp. Ex. 4 | Cu/Ni/Pd | 4.0 | 4.3 |

Note: * catalyst composition Cu/Ni/Pd/Ni = 4/1/0.05/0.05

Cu/Ni = 4/1

Cu/Ni/Pd = 4/1/0.05

It was found from the results that when the Cu/Ni/platinum group element (Pd)/fourth component (Li) quaternary catalyst system according to the present invention was used, the hue of the quaternary ammonium salt was far better than that of the quaternary ammonium salt in the case where the Cu/Ni binary catalyst system (Comparative Example 3) and the Cu/Ni/Pd ternary catalyst system (Comparative Example 4) were used.

Examples 3 to 7

With respect to a catalyst comprising copper, a fourth period transition metal element, a platinum group element, and a fourth component, the activity n a reaction of stearyl alcohol with monomethylamine was determined by using Cr as the fourth period transition metal element and Ru as the platinum group element and varying the kind of the fourth component among Li, Na, K, Rb, and Cs in the catalyst. These quaternary catalysts were prepared in the same manner as that of Example 1. Further the tertiary amine thus formed was purified by distillation and then reacted with methyl chloride, followed by observation of the hue (Lovibond Red) of the resultant quaternary salt.

The results are shown in Table 3.

It was found from the results that when distearylmonomethyl tertiary amine was prepared through a reaction of stearyl alcohol with monomethylamine, the Cu/Cr/Ru/fourth component catalysts wherein Li, Na, K, Rb, or Cs is used as the fourth component exhibited an excellent activity, brought about an improvement in the selectivity, and contributed to an improvement in the hue of the quaternary ammonium salt.

Table 3

| Ex. | catalyst fourth component metal* | reaction time (hr) | composition of reaction product (wt%) | | | hue of distearyldimethyl-ammonium chloride (Lovibond Red) |
|---|---|---|---|---|---|---|
| | | | unreacted alcohol | distearylmono-methylamine | Others | |
| 3 | Li | 5 | 1.4 | 97.6 | 1.0 | 0.6 |
| 4 | Na | 5 | 1.0 | 98.2 | 0.8 | 0.7 |
| 5 | K | 5 | 0.8 | 97.8 | 1.4 | 0.5 |
| 6 | Rb | 5 | 1.3 | 97.4 | 1.3 | 0.6 |
| 7 | Cs | 5 | 1.7 | 97.6 | 0.7 | 0.5 |

Note: Cu/Cr/Ru/fourth component: molar ratio 3/1/0.03/0.01

amount of support 50%

reaction conditions:

alcohol: stearyl alcohol

amine: monomethylamine

temperature: 200°C

amount of addition of catalyst: 0.25% based on alcohol

Examples 8 to 11

With respect to a catalyst comprising copper, a fourth period transition metal element, a platinum group element, and a fourth component, the activity in a reaction of dodecyl alcohol with ammonia was

8

EP 0 489 722 B1

determined by using Zn as the fourth period transition metal element and Pt as the platinum group element and varying the kind of the fourth component among Mg, Ca, Sr, and Ba in the catalyst. These quaternary catalysts were prepared in the same manner as that of Example 1.

The results are shown in Table 4.

It was found from the results that when tridodecylamine was to be prepared through a reaction of dodecyl alcohol with ammonia, the Cu/Zn/Pt/fourth component catalysts wherein Mg, Ca, Sr, or Ba is used as the fourth component provided an excellent activity and improved in selectivity.

## Table 4

| | | catalyst * fourth component metal | reaction time (hr) | composition of reaction product (wt%) | | |
|---|---|---|---|---|---|---|
| | | | | unreacted alcohol | tridodecyl-amine | Others |
| Ex. | 8 | Mg | 10 | 0.8 | 98.2 | 1.0 |
| | 9 | Ca | 10 | 1.0 | 97.5 | 1.5 |
| | 10 | Sr | 10 | 1.3 | 97.9 | 0.8 |
| | 11 | Ba | 10 | 1.1 | 98.2 | 0.7 |

Note:    Cu/Zn/Pt/fourth component:   molar ratio 5/1/0.05/0.5

amount of support 50%

reaction conditions:

alcohol:                          dodecyl alcohol

amine:                            ammonia

amine feed rate:                  10 ℓ/hr

reaction temperature:             180°C

amount of addition of catalyst:  1.0% based on alcohol

Examples 12

A reaction of lauryl alcohol with ammonia was conducted in the presence of a Cu/Co/Pd/fourth component (Ba) catalyst. In this reaction, ammonia was blown into the system at a feed rate of 30 ℓ/hr. The reaction was monitored by measurement of an amine value and gas chromatography. The secondary amine thus prepared was purified by distillation and then reacted with benzyl chloride, followed by observation of the hue (Lovibond Red) of the resultant quaternary salt.

The results are shown in Table 5.

It was found from the results that the use of the Cu/Co/Pd/fourth component (Ba) catalyst in a reaction of lauryl alcohol with ammonia enabled the preparation of a secondary amine with a high activity and brought about a remarkable improvement in the hue of the quaternary ammonium salt.

Table 5

| catalyst composition* | reaction time (hr) | composition of reaction product (wt%) | | | hue of dilauryl-methylbenzylammonium chloride (Lovibond Red) |
|---|---|---|---|---|---|
| | | dilauryl-amine | tertiary amine | Others | |
| Ex. 12 | Cu/Co/Pd/Ba | 5 | 94.1 | 2.0 | 3.9 | 0.6 |

Note:  Cu/Co/Pd/Ba:  molar ratio 3/1/0.03/0.5
amount of support 40%

reaction conditions:

alcohol:  lauryl alcohol

amine:  ammonia

amine feed rate:  30 ℓ/hr

reaction temperature:  180°C

amount of addition of catalyst:  0.25% based on alcohol

Example 13

A reaction of lauryl alcohol with stearylamine was conducted in the presence of a Cu/Mn/Ru catalyst. In this reaction, the stearylamine was introduced at a time in a liquid state into the reaction system. The reaction was monitored by measurement of an amine value and gas chromatography.

The results are shown in Table 6.

It was found from the results that the present catalyst system enabled the reaction to proceed with very high activity and the corresponding amine to be prepared with a high selectivity.

Table 6

| | catalyst * composition | reaction time (hr) | composition of reaction product (wt%) | | |
|---|---|---|---|---|---|
| | | | unreacted alcohol | N-lauryl-stearyl-amine | Others |
| Ex. 13 | Cu/Mn/Ru/Ca | 5 | 2.3 | 89.0 | 8.7 |

Note: * Cu/Mn/Ru/Ca:  molar ratio        4/1/0.1/0.3

amount of support   20%

reaction conditions:

    alcohol:                                    lauryl alcohol

    amine:                                      stearylamine

    alcohol/amine = 1

    reaction temperature:                       180°C

    amount of addition of catalyst:   1.0% based on alcohol

Examples 14 to 17

The effect of the catalyst of the present invention was examined with respect to reactions of various alcohols or aldehydes with dimethylamine to prepare the corresponding tertiary amines. The catalysts were prepared by the impregnation process.

The results are shown in Table 7.

Table 7

| Ex. | alcohol or aldehyde | catalyst *3 | | reaction temp. (°C) | reaction time (hr) | composition (wt%) *4 | | |
|---|---|---|---|---|---|---|---|---|
| | | fourth component | amount of addition (%) | | | tertiary amine | unreacted alcohol/ aldehyde | Others |
| 14 | oxo alcohol *1 | Mg | 0.5 | 200 | 7 | 95.3 | 2.0 | 2.7 |
| 15 | Guerbet alcohol *2 | Ca | 0.5 | 230 | 7 | 85.6 | 10.2 | 4.2 |
| 16 | 1,6-hexanediol | Sr | 0.5 | 180 | 7 | 95.6 | 2.3 | 2.1 |
| 17 | lauraldehyde | Br | 0.5 | 180 | 7 | 96.3 | 0.5 | 3.2 |

Note:  *1 oxo alcohol: a mixture of alcohols having 12 to 13 carbon atoms; a degree of branching of 21% (straight-chain alcohol: 79%)

*2 Guerbet alcohol: $\begin{array}{c} R' \\ R'' \end{array}$ CH-CH$_2$OH (total of carbon atoms: 28)

*3 Cu/Ni/Ru/fourth component molar ratio: 5/1/0.02/0.05, amunt of addition: based on alcohol or aldehyde.

*4 The tertiary amine is monoalkyldimethyl tertiary amine (provided that the tertiary amines in the case of 1,6-hexanediol and lauraldehyde are N,N,N'N'-tetramethyl-hexamethylenediamine and lauryldimethyl tertiary amine, respectively).

It was found from the above results that the catalyst of the present invention enabled a tertiary amine to be prepared with very high activity and high selectivity even when a branched alcohol, a polyhydric alcohol (glycol), or an aldehyde was used as the starting material and reacted with a secondary amine.

In general, the use of such a branched alcohol, a polyhydric alcohol, or an aldehyde as the starting material brings about an increase in the possibilities of causing side reactions such as decomposition or condensation of these substances. However, it was substantiated that the catalyst having a composition according to the present invention is an excellent catalyst capable of solving these problems.

Examples 18

A reaction of lauryl alcohol with stearylamine was conducted in the presence of a Cu/Fe/Pd/fourth component (K) catalyst. In this reaction, stearylamine was introduced at a time in a liquid state into the reaction system. The reaction was monitored by measurement of an amine value and gas chromatography. The reaction pressure was 50 atm (gauge).

The results are shown in Table 8.

It was found from the results that the present catalyst system enabled the preparation of an amine through a reaction of lauryl alcohol with stearylamine with a high activity and a high selectivity.

## Table 8

| | catalyst composition * | reaction time (hr) | composition of reaction product (wt%) | | |
| --- | --- | --- | --- | --- | --- |
| | | | unreacted alcohol | N-lauryl-stearyl-amine | Others |
| Ex. 18 | Cu/Fe/Pd/K | 5 | 0.3 | 90.4 | 9.3 |

Note: * Cu/Fe/Pd/K: molar ratio          6/1/0.01/0.8

              amount of support   20%

reaction conditions

        alcohol:                      lauryl alcohol

        amine:                        stearylamine

        alcohol/amine = 1

        reaction temperature:         160°C

        amount of addition of catalyst: 1.0% based on alcohol

Example 19

The reaction mixture prepared in Example 1 was filtered to recover the catalyst therefrom, and the amination reaction was repeatedly conducted under the same conditions.

The results are shown in Table 9.

Table 9

| number of runs | reaction time (hr) | composition of reaction product (wt%) | | |
|---|---|---|---|---|
| | | unreacted alcohol | lauryldimethylamine | Others |
| 1 | 5 | 1.6 | 97.6 | 9.8 |
| 2 | 5 | 1.5 | 97.5 | 1.0 |
| 3 | 5 | 1.7 | 96.3 | 2.0 |
| 4 | 5 | 1.5 | 96.7 | 1.8 |
| 5 | 5 | 1.9 | 96.5 | 1.6 |

Example 20

Behenyl alcohol and stearylamine, fed at a molar ratio of 1/1, were reacted with each other in the presence of 2 percent by weight, based on the alcohol, of a catalyst of Cu, Mn, Rh and K, having a molar ratio of 95/5/0.05/0.5 and an amount of support of 40 percent by weight, at 200°c. The stearylamine was introduced at a time in the form of liquid into the reaction system and the reaction procedure was followed with an amine value and gas chromatographic analysis. Results are shown in Table 10. It is found that a corresponding amine can be produced with a high reactivity and a high selectivity from a long chain alcohol and a long chain amine, with the catalyst of the invention.

Table 10

| example 20 | |
|---|---|
| catalyst<br>reaction time (hour) | Cu/Mn/Rh/K<br>5 |
| composition of product mixture (weight percent) | |
| unreacted alcohol<br>N-stearyl-behenylamine<br>others | 1.8<br>91.2<br>7.0 |

**Claims**

1. A process for preparing an N-substituted amine, which comprises the step of reacting an alcohol or an aldehyde with ammonia, a primary amine or a secondary amine at a temperature from 150 to 250°C and at a pressure from atmospheric pressure to 100 atm gauge, with removal of water formed in the reaction, in the presence of a catalyst comprising (1) copper, (2) a metal selected from chromium, manganese, iron, cobalt, nickel and zinc, (3) a metal of the platinum VIII group an (4) a metal selected from alkali metals and alkaline earth metals.

2. A process as claimed in claim 1, in which the molar ratio of the component (1) to (2) ranges from 10/90 to 99/1, the molar ratio of the component (3) to the sum total of (1) and (2) ranges from 0.001 to 0.1 and the molar ratio of the component (2) and (4) ranges from 1/0.01 to 1/1.

**Patentansprüche**

1. Verfahren zur Herstellung eines N-substituierten Amins, umfassend den Schritt des Umsetzens eines Alkohols oder eines Aldehyds mit Ammoniak, einem primären Amin oder einem sekundären Amin bei einer Temperatur von 150 bis 250°C bei einem Druck von Atmosphärendruck bis 100 Atmosphären Manometeranzeige, bei Entfernung des in der Umsetzung gebildeten Wassers, in Gegenwart eines

14

Katalysators, umfassend (1) Kupfer, (2) ein Metall, ausgewählt aus Chrom, Mangan, Eisen, Kobalt, Nickel und Zink, (3) ein Metall der Platingruppe VIII und (4) ein Metall, ausgewählt aus Alkalimetallen und Erdalkalimetallen.

2. Verfahren nach Anspruch 1, worin das molare Verhältnis der Komponente (1) zu (2) im Bereich von 10/90 bis 99/1, das molare Verhältnis der Komponente (3) zu der Gesamtsumme von (1) und (2) im Bereich von 0,001 bis 0,1 und das molare Verhältnis der Komponente (2) und (4) im Bereich von 1/0,01 bis 1/1 liegt.

**Revendications**

1. Procédé pour la préparation d'une amine N-substituée, comprenant l'étape consistant à faire réagir un alcool ou un aldéhyde avec de l'ammoniaque, une amine primaire ou une amine secondaire à une température comprise entre 150 et 250°C et à une pression manométrique comprise entre la pression atmosphérique et 100 atmosphères, en présence d'un catalyseur comprenant (1) du cuivre, (2) un métal choisi parmi le chrome, le manganèse, le fer, le cobalt, le nickel et le zinc, (3) un métal du groupe VIII du platine, puis (4) un métal choisi parmi les métaux alcalins et les métaux alcalino-terreux, tout en éliminant l'eau formée en cours de réaction.

2. Procédé selon la revendication 1, dans lequel le rapport molaire du composé (1) au composé (2) varie entre 10/90 et 99/1, le rapport molaire du composé (3) à la somme des composés (1) et (2) varie entre 0,001 et 0,1 et le rapport molaire du composé (2) au composé (4) varie entre 1/0,01 et 1/1.

15